Europäisches Patentamt

European Patent Office

Office européen des brevets

(11) Numéro de publication: **0 213 405 B1**

**FASCICULE DE BREVET EUROPEEN**

(45) Date de publication du fascicule du brevet: **27.06.90**

(51) Int. Cl.⁵: **A 01 K 67/00**

(21) Numéro de dépôt: **86110569.0**

(22) Date de dépôt: **31.07.86**

(54) **Procédé et dispositif de production en masse d'un insecte entomophage, son application en lutte biologique.**

(30) Priorité: **07.08.85 FR 8512115**

(43) Date de publication de la demande: **11.03.87 Bulletin 87/11**

(45) Mention de la délivrance du brevet: **27.06.90 Bulletin 90/26**

(84) Etats contractants désignés: **AT BE CH DE GB IT LI LU NL SE**

(56) Documents cités: **FR-A-2 202 644**

(73) Titulaire: **Société coopérative agricole dite: SOCIETE COOPERATIVE AGRICOLE DE SEMENCES DE LIMAGNE" LIMAGRAIN" Chappes Boite Postale No. 51 F-63360 Gerzat (Puy-de-Dôme) (FR)**

(73) Titulaire: **Union de coopératives agricoles à personnel et capital variables dite: UNION NATIONALE DES COOPERATIVES AGRICOLES D'APPROVISIONNEMENT 83-85 avenue de la Grande Armée F-75016 Paris (FR)**

(73) Titulaire: **Etablissement public dit: INSTITUT NATIONAL DE LA RECHERCHE AGRONOMIQUE 149, rue de Grenelle F-75341 Paris Cedex 07 (FR)**

(72) Inventeur: **Pizzol, Jeannine, née Dalmasso Chemin des Eucalyptus Parc des Mimosas F-06160 Juan-Les-Pins (Alpes Maritimes) (FR)**
Inventeur: **Voegele, Jean 428 Chemin des Rastines F-06600 Antibes (Alpes Maritimes) (FR)**
Inventeur: **Jourdheuil, Pierre Lotissement Volmare Les Rastines F-06600 Antibes (Alpes Maritimes) (FR)**

Courier Press, Leamington Spa, England.

# EP 0 213 405 B1

(72) Inventeur: **Raynaud, Bernard**
**8 rue Marcel et Georgette Sambat**
**F-94500 Champigny-sur-Marne (FR)**
Inventeur: **Miermont, Yves**
**5 rue Baracane Résidence Les Célestins**
**F-84000 Avignon (Vaucluse) (FR)**

(74) Mandataire: **Simonnot, Bernard et al**
**Cabinet Simonnot 35 rue de Clichy**
**F-75442 Paris Cédex 09 (FR)**

# EP 0 213 405 B1

**Description**

La présente invention concerne de façon générale la multiplication et la production en masse d'un insecte entomophage tel que Trichogramma maïdis, à partir d'un hôte de substitution tel que l'oeuf de la Pyrale de la farine Ephestia kuehniella, ledit entomophage étant dans ce cas utilisé en lachers inondatifs saisonniers dans le cadre de la lutte biologique contre un ravageur des cultures de maïs, Ostrinia nubilalis ou Pyrale du maïs.

L'invention concerne donc plus particulièrement un procédé de production en masse d'un insecte entomophage, avec un dispositif pour sa mise en oeuvre, ainsi que l'application dudit procédé en lutte biologique au moyen de micro-conteneurs destinés au laucher au champ.

Dans le cas du Trichogramme, la production de cet entomophage se réalise, selon FR—A—2 202 644 dans des chambres dites "climatiques" où des oeufs hôtes sont présentés en monocouche sur plaques à une quantité de Trichogrammes adultes, ou inoculum, dans un rapport déterminé en fonction du Trichogramme choisi, et précédemment émergés d'oeufs parasités disposés en plaques dans la chambre.

Lorsqu'il est nécessaire de procéder à un stockage au froid avant utilisation des oeufs parasités, la durée de parasitisme des oeufs hôtes par l'inoculum de Trichogrammes doit être limitée à 48 heures à 25°C (cf. travaux de Madame PIZZOL sur l'induction de la diapause chez Trichogramma maïdis permettant ce stockage au froid). En moyenne 75% des oeufs hôtes ainsi présentés sont parasités. Une partie des oeufs ainsi parasités est toutefois recyclée comme inoculum pour la génération suivant.

Ce procédé de multiplication en cages pendant 24 à 48 heures présente certains inconvénients:

1 — manipulation de nombreuses cages sans grande possibilité d'automatisation;

2 — utilisation partielle du potentiel de ponte des femelles Trichogrammes, le parasitisme étant au plus de 48 heures à 25°C, condition minimale indispensable à un taux correct de mise en induction de diapause des larves de Trichogrammes avant leur stockage au froid.

Le temps de parasitisme de la partie recyclée comme inoculum ne doit pas non plus dépasser ces 48 heures pour éviter un trop grand étalement des émergences dans les cages. Même dans ce cas, il est alors nécessaire d'attendre au moins 48 heures après les premières émergences de l'inoculum pour introduire la plaque d'oeufs hôtes.

La présente invention vise donc à remédier à ces inconvénients, en fournissant notamment un procédé de multiplication et production en masse de'insectes d'entomophages par formation d'oeufs hôtes parasités en cage de population, trouvant une application avantageuse dans le cas du Trichogramme et de la lutte biologique contre la Pyrale du maïs.

La solution selon la présente invention consiste en un procédé de production en masse d'un insecte entomophage, en cage de multiplication où des insectes adultes provenant d'un inoculum précédemment constitué formé de premiers oeufs-hôtes parasités et disposés sur un premier support, sont mis en présence de seconds oeufs-hôtes à parasiter disposés sur un second support dont on prélève une portion pour former ledit inoculum, procédé caractérisé par le fait que l'on maintient à température constante une population constante d'insectes adultes à partir dudit inoculum auquel on a fait subir une incubation préalable, que l'on active ledit inoculum dans la cage pendant une durée correspondant au temps nécessaire à l'émergence de l'adulte à ladite température, que l'on fait défiler en continu à la même vitesse dans ladite cage lesdits premier et second supports, ladite vitesse permettant de présenter de façon continue et régulière pendant une durée de présentation déterminée les seconds oeufs-hôtes devant être parasités par l'insecte ayant émergé dudit inoculum, tout en alimentant ledit insecte, puis que l'on recueille en continu les seconds oeufs-hôtes ainsi parasités dont on prélève la fraction de constitution de l'inoculum sous la forme dudit premier support après l'incubation préalable.

Après avoir recueilli les seconds oeufs hôtes parasités, on les applique en tant qu'inoculum en présence d'oeufs sains de l'entomophage, sous forme d'un mélange dosé introduit en micro-conteneur destiné au lacher au champ et traité en fonction de l'étalement d'émergence des insectes entomophages désiré.

Pour la mise en oeuvre du procédé tel que défini ci-dessus, le dispositif comprend en enceinte éclairée et régulée en température constituant une cage de population, et est caractérisé par le fait que le premier suppport destiné à l'inoculum est constitué par une bande transporteuse étroite, le second support destiné aux oeufs-hôtes à parasiter étant constitué par une bande transporteuse large, que la cage comprend une entrée de ladite bande large et un logement de mise en activation de l'inoculum, ainsi qu'une sortie de ladite bande large et d'enroulement d'une spire étroite destinée à la formation dudit inoculum, lesdites bandes transporteuses étant guidées sur des rouleaux et entraînées en continu à la même vitesse par un groupemoteur ledit logement de mise en activation pouvant contenir des spires étroites pendant la durée d'activation de l'inoculum et que ladite cage est en outre associée à des moyens extérieurs de récupération des oeufs-hôtes parasités et de reconditionnement de ladite bande large.

D'autres caractéristiques et avantages de l'invention ressortiront mieux de la description qui va suivre, faite en regard des dessins annexés sur lesquels:

la figure 1 représente un schéma synoptique du dispositif selon l'invention, illustrant le procédé;

la figure 2 représente une vue schématique de l'avant de la cage de population;

la figure 3 représente une vue schématique de l'ensemble général d'implantation du dispositif de production;

3

# EP 0 213 405 B1

la figure 4 représente une vue schématique à échelle agrandie d'un détail des rouleaux de guidage des bandes transporteuses;

la figure 5 représente un schéma synoptique du mode de parasitisme en capsule;

les figures 6 et 7 représentent des graphiques illustrant l'étalement des émergences au lacher, en fonction du mode de parasitisme.

Sur ces dessins, les mêmes références désignent les mêmes éléments.

Dans le cas par exemple du Trichogramme, on maintient dans une cage une population desdits Trichogrammes, constante de par sa quantité et ses potentialités biologiques, et l'on amène de façon continue et régulière les oeufs hôtes en vue de leur parasitisme. A cet effet, un apport régulier de Trichogrammes compensant la mortalité est nécessaire et fourni par la cage elle-même, sous forme de premiers oeufs hôtes parasités et incubés deux semaines à 22°C, introduits dans un logement prévu à cet effet sur la cage où les Trichogrammes vont pouvoir émerger et parasiter les seconds oeufs hôtes qui leur sont présentés en continu. De manière avantageuse, le temps de présence dans la cage de l'inoculum est de jours et celui des seconds oeufs d'hôtes est de 24 heures, comme ce sera mieux expliqué ci-après.

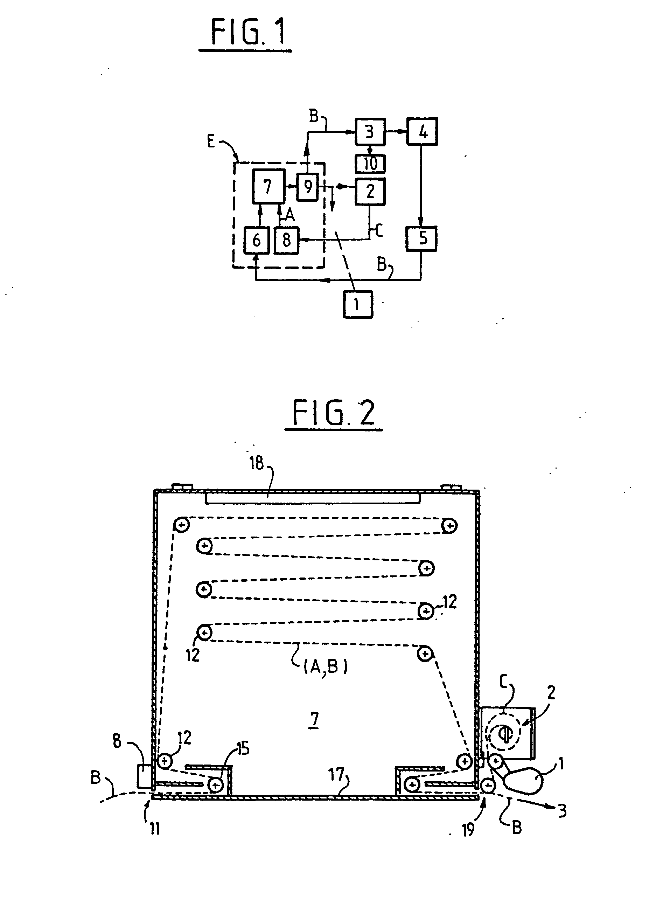

En se référant à la figure 1, le dispositif pour la mise en oeuvre du procédé ci-dessus comprend différents postes par rapport à une unité de référence generale E comprenant la cage proprement dite 7 dans laquelle défilent en continu deux bandes transporteuses A et B, respectivement étroite pour les premiers oeufs hôtes constituant l'inoculum-cage et large à double face pour les seconds oeufs hôtes, toutes deux entraînées à la vitesse convenable par un groupe-moteur 1.

Sur la sortie de la cage de population 7, une bande étroite C supportant des oeufs venant d'être parasités et destinés à la formation de l'inoculum-cage est enroulée grâce au moteur d'entraînement 1 en une spire de référence générale 2.

Pour l'activation de cet inoculum-cage, après l'incubation, les spires sont introduites en 8 dans la cage, dans un logement où cinq spires 2 tiennent place. L'introduction d'une spire 2 en 8 provoque l'expulsion d'une spire 2 en 9 hors de la cage. Une spire 2 demeure donc cinq jours dans la cage, ce qui convient pour une émergence complète des Trichogrammes à partir des premiers oeufs hôtes.

A la sortie de la cage de population 7, la bande large B supportant par encollage les seconds oeufs hôtes parasités est brossée en 3 sur ses deux faces, le oeufs étant récupérés en 10 sous forme de poudre pour leur traitement ultérieur. Une fois brossée, la bande B est nettoyée en 4, rincée, puis de nouveau encollée et garnie d'oeufs hôtes sains sur ses deux faces en 5 avant sa réintroduction en 6 dans la cage 7. Le cheminement entre les postes 3 et 6 extérieurs à la cage 7 dure également 24 heures.

En se référant aux figures 1 à 4, la cage 7 est constituée par une enceinte munie d'une entrée 11 représentant les deux postes 6 et 8, respectivement pour la bande B et pour la bande A. Ces deux bandes sont guidées sur des rouleaux coaxiaux 12, l'un pour la bande A (13) et l'autre pour la bande B (14). Le premier rouleau 15 de l'entrée 11 est avantageusement muni d'un frein usuel 16 destiné à régler la tension des bandes. La cage comporte en outre un fond mobile 17, destiné en particulier à éliminer les Trichogrammes morts. Par ailleurs, une source de lumière appropriée 18 est disposée sur la face interne supérieure de la cage 7 afin de maintenir l'essentiel de la population de Trichogrammes vers le haut de la cage, tandis qu'on alimente ces derniers par exemple au moyen de miel pouvant être disposé soit automatiquement sur le bord des bandes, soit manuellement au niveau de ladite face interne supérieure.

Comme mentionné plus haut, les bandes A et B sont entraînées par un groupe-moteur 1, à une vitesse telle que ces bandes parcourent leur trajet à la même vitesse entre l'entrée 11 et la sortie 19 de la cage en 24 heures. A la sortie 19, la bande A est extraite, pendant qu'une nouvelle bande d'oeufs parasités qui ont été soumis à l'incubation dans un poste séparé (non représenté) est introduite sous forme d'une spire 20 au poste 8. La bande B est dirigée de son côté sur un rouleau à brosses 3 d'où les seconds oeufs hôtes, parasités, sont recueillis sous forme de poudre dans un réceptacle 10. La bande B est ensuite rincée dans un bain 4, ré-encollée en 5 sur ses deux faces et garnie en oeufs hôtes sains, puis introduite dans la cage par l'entrée 11. La bande A, provenant du poste d'incubation, introduite sous forme d'une spire 20 dans un des logements 8 de la cage 7, progresse pour son activation à une vitesse telle que son trajet total s'effectue en cinq jours, durée correspondant au temps nécessaire à l'émergence des Trichogrammes.

Conformément à la présente invention, on applique ensuite les oeufs hôtes recueillis dans le réceptacle 10 du dispositif de production, en tant qu'inoculum mis en présence de troisièmes oeufs hôtes sains dans des micro-conteneurs, chacun sous forme par exemple d'une capsule en carton-pâte de 1,2 cm de diamètre en deux demi-sphères collées puis ultérieurement percées et destinées au lacher au champ.

Le principe consiste à faire parasiter les oeufs hôtes directement dans cette capsule en y associant l'inoculum-capsule approprié. Celui-ci est calculé de telle sorte que le nombre d'oeufs parasités soit maximal tout en limitant le superparasitisme à un seuil acceptable. Dans le cas des capsules, le rapport oeufs "noirs" parasités (inoculum)/oeufs "blancs" à parasiter est de 1/15.

Suivant que les sorties des adultes sont groupées ou étalées, on obtient, dans le premier cas, la possibilité de parasiter avec un inoculum donné le plus grand nombre d'oeufs hôtes dans un court délai, alors que dans le second cas, on prolonge la période d'action des Trichogrammes au champ.

Le regroupement des sorties est par ailleurs indispensable dans le cas du conditionnement du développement du Trichogramme au froid pour l'induction d'une diapause autorisant le stockage de longue durée du produit biologique, de la manière décrite dans le brevet français n° 79 17 774. Afin de définir les conditions optimales de traitement et de chargement des capsules, on a soumis des lots d'oeufs

4

parasités, où apparaissait le tout premier adulte, à des températures relativement basses (10, 14 et 18°C et pendant des durées de 24 à 48 heures) de manière à freiner le développement nymphal et regrouper la sortie des adultes. Après ce traitement au froid, ces lots sont exposés à des températures élevées (22 à 25°C avec contrôles matin et soir des sorties durant trois jours) pour l'obtention des sorties des Trichogrammes adultes dans un court délai. Un lot témoin a été soumis par ailleurs à un développement à une température constante de 22°C.

Les résultats regroupés dans le tableau I cidessous montrent que ce sont les inoculum exposés à 10° et à 14°C pendant 24 à 48 heures et soumis à un passage à 25°C pendant 15 heures, qui offrent les conditions optimales de sorties (>90%).

Tableau I : Taux d'émergence ou de sorties avant ou après réchauffement à 25ºC
et 22ºC en fonction de durées de 24 et 48 heures d'exposition à
des températures proches du seuil de développement.

| Traitement au froid | | Taux d'émergence avant réchauffement | Taux d'émergence après réchauffement à 25°C | | | | | | Taux d'émergence avant réactivation | Taux d'émergence après réchauffement à 22° C | | | | | |
|---|---|---|---|---|---|---|---|---|---|---|---|---|---|---|---|
| | | | 1er jour | | 2ème jour | | 3ème jour | | | 1er jour | | 2ème jour | | 3ème jour | |
| Tempeature | Durée | | matin | soir | matin | soir | matin | soir | | matin | soir | matin | soir | matin | soir |
| 10°C | 24 H | 2% | | | | | | | 2% | ≤ 90% | | | | | |
| | 48 H | | | | | | | | | | | | | | |
| 14°C | 24 H | à | > 90% | | > 90 % | | | | à | < 80% | > 90 %· | | | | |
| | 48 H | 8% | | | | | | | 8% | | | | | | |
| 18°C | 24 H | 30 % | | | | | | | 30% | ≤ 87% | | | | | |
| | 48 H | à 41 % | | | | | | | à 41 % | | | | | | |
| Témoin 22° C | | | 6,2 % | | 16,8 % | | 86% | | | | | | | | |

EP 0 213 405 B1

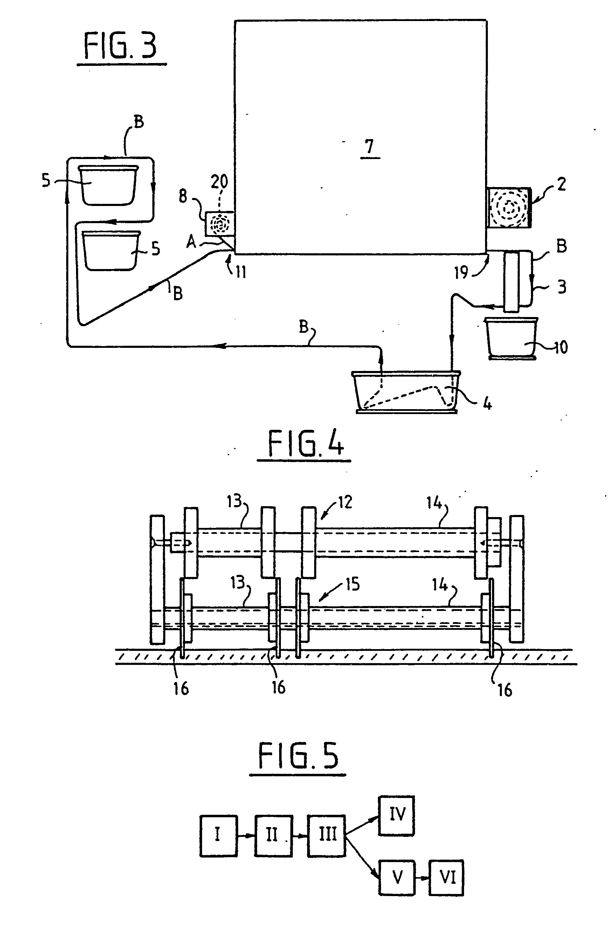

En conséquence et de manière pratique, on introduit dans les capsules les oeufs hôtes à parasiter ainsi que l'inoculum. Ces capsules, une fois fermées, sont exposées à 10 ou 14°C durant 24 à 48 heures avant d'être soumises à 25°C pour une période de 15 heures. Après ce séjour à 25°C on peut obtenir deux types de développement: Un développement continu en soumettant les capsules à 22—25°C jusqu'à la mort des Trichogrammes, ou un arrêt de développement dit diapause en soumettant les capsules seulement de 24 à 36 heures à 22—25°C et en les exposant ensuite quarante jours à 14°C puis trois à douze mois à 3°C. Les différentes phases de traitement sont reprises dans le schéma synoptique de la figure 5, àsavoir:

I: Mise en capsule — Inoculum avec premier adulte sortie + oeufs hôtes d'Ephestia kuehniella à parasiter.

II: Ralentissement du développement au froid: 10—14°C 24 à 48 heures.

III: Réchauffement pour regrouper la sortie des adultes: 25°C, 15 heures.

IV: Développement continu: 22 ou 25°C.

V: Développement 22 ou 25°C, 24 à 36 heures.

VI: Diapause — Induction quarante jours à 14°C; stockage trois à douze mois à 3°C.

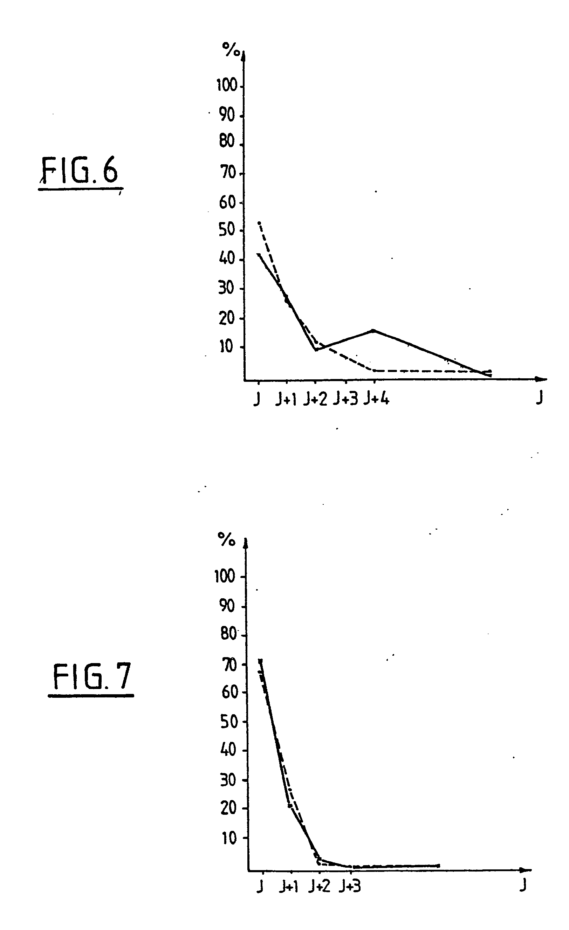

L'étalement des éclosions de la progéniture issue de cet inoculum a été suivi à la fois pour le développement continu et le développement après stockage à 3°C pendant trois mois. Les résultants sont illustrés sur les graphiques des figures 6 et 7 où l'on a porté en abscisses l'étalement des émergences en jours et, en ordonnées, le pourcentage d'adultes émergés. Sur ces graphiques, les courbes en trait continu conernent des capsules percées de petits orifices, tandis que les courbes en pointillés concernent des capsules ouvertes.

Dans le premier cas, il y à étalement des sorties pendant cinq jours avec environ 75% des sorties dans les premières 48 heures: environ 48% le premier jour, environ 27% le deuxième jour (Fig. 6) et encore 20% des sorties du troisième au cinquième jour.

Dans le deuxième cas, on obtient 95% des sorties dans les premières 48 heures, avec environ 70% le premier jour et 25% le deuxième jour (Fig. 7).

Le mode de parasitisme décrit ci-dessus permet en capsule un rapport inoculum-oeufs sains de l'hôte au moins égal à 1/15 avec superparasitisme négligeable, d'où une économie substantielle d'inoculum-capsule. En outre, l'ensemble de la présente invention permet d'obtenir une chaîne automatisée de production, depuis l'inoculum de base jusqu'a la capsule de lacher au champ, ladite capsule pouvant en outre produire des insectes entomophages à émergence groupée ou étalée, tout en assurant une possibilité de stockage d'éléments conditionnés prêts à l'emploi.

Comme mentionné plus haut, les capsules nécessitent naturellement d'être percées pour permettre la libération des insectes émergés, au moyen par exemple d'un dispositif à aiguilles automatique. Le perçage doit toutefois être effectué en fonction de la modalité d'utilisation adoptée, à savoir, soit juste avant le traitement à 10—14°C, soit après le stockage à 3°C faisant suite à la diapause.

La description détaillée donnée ci-dessus dans le cadre du Trichogramma maïdis peut naturellement s'appliquer pour la production d'un autre insecte, puiqu'il suffit alors d'adapter les conditions de températures et de durées aux caractéristiques biologiques de l'individu considéré.

Il est d'ailleurs bien entendu que la présente invention n'a été décrite et représentée qu'a titre explicatif mais nullement limitatif et qu'on pourra y apporter toute modification utile, notamment dans le domaine des équivalences techniques, sans sortir du cadre délimité par les revendications.

**Revendications**

1. Procédé de production en masse d'un insecte entomophage, en cage de multiplication où des insectes adultes provenant d'un inoculum précédemment constitué formé de premiers oeufs-hôtes parasités et disposés sur un premier support, sont mis en présence de seconds oeufs-hôtes à parasiter disposés sur un second support dont on prélève une portion pour former ledit inoculum, procédé caractérisé par le fait que l'on maintient à température constante une population constante d'insectes adultes à partir dudit inoculum auquel on a fait subir une incubation préalable, que l'on active ledit inoculum dans la cage pendant une durée correspondant au temps nécessaire à l'émergence de l'adulte à ladite température, que l'on fait défiler en continu à la même vitesse dans ladite cage lesdits premier et second supports, ladite vitesse permettant de présenter de façon continue et régulière pendant une durée de présentation déterminée les seconds oeufs-hôtes devant être parasités par l'insecte ayant émergé dudit inoculum, tout en alimentant ledit insecte, puis que l'on recueille en continu les seconds oeufs-hôtes ainsi parasités dont on prélève la fraction de constitution de l'inoculum sous la forme dudit premier support après l'incubation préalable.

2. Procédé selon la revendication 1, caractérisé par le fait que l'insecte entomophage est le Trichogramma maïdis et l'oeuf hôte est celui de Ephestia kuehniella, la température de la cage étant de 22°C, le temps de présentation étant de 24 heures et le temps d'activation de l'inoculum étant de cinq jours.

3. Dispositif pour la mise en oeuvre du procédé selon l'une des revendications 1 ou 2, comprenant une enceinte éclairée et régulée en température constituant une cage de population (7), caractérisé par le fait que le premier support destiné à l'inoculum est constitué par une bande transporteuse étroite (A), le second support destiné aux oeufs-hôtes à parasiter étant constitué par une bande transporteuse large (B), que la cage comprend une entrée (11) de ladite bande large (B) et un logement (8) de mise en activation de l'inoculum (A), ainsi qu'une sortie (19) de ladite bande large (B) et d'enroulement d'une spire étroite (2) destinée à la formation dudit inoculum, lesdites bandes transporteuses étant guidées sur des rouleaux (12) et entraînees en continu à la même vitesse par un groupemoteur (1) ledit logement (8) de mise en activation pouvant contenir des spires étroites (2) pendant la durée d'activation de l'inoculum et que ladite cage est une outre associée à des moyens extérieurs de récupération (10) des oeufs-hôtes parasités et de reconditionnement (4, 5) de ladite bande large (B).

4. Dispositif selon la revendication 3, caractérisé par le fait que les rouleaux (2) sont constitués de deux rouleaux coaxiaux, l'un (13) pour la bande (A), étroite, de l'inoculum, l'autre (14) pour la bande (B), plus large, des seconds oeufs hôtes à parasiter.

5. Dispositif selon l'une des revendications 3 ou 4, caractérisé par le fait que le moyen de récupération des oeufs hôtes parasités est constitué par un rouleau à brosse (10) recevant la bande (B), et que les moyens de reconditionnement de ladite bande comprennent un organe de rinçage (4) et un organe d'encollage (5) sur ses deux faces d'oeufs hôtes sains, dont la sortie est reliée à l'entrée (11) de la cage, la durée du trajet de ladite bande à l'extérieur de la cage étant de 24 heures.

6. Dispositif selon la revendication 5, caractérisé par le fait que le logement (8) de la cage contient cinq spires (2) de support de l'inoculum ayant subi l'incubation préalable et destinées séjourner chacune cinq jours dans la cage avec renouvellement d'une spire toutes les 24 heures.

7. Application du procédé selon l'une des revendications 1 ou 2 à la lutte biologique par lacher au champ de capsules renfermant des oeufs d'insecte entomophage, caractérisé par le fait qu'on met en oeuvre les seconds oeufs hôtes parasites obtenus par le procédé selon la revendication 1 ou 2, en tant qu'inoculum en présence d'oeufs hôtes sains à l'intérieur de capsules, dans le rapport 1/15.

8. Application selon la revendication 7, caractérisée par le fait que l'on soumet les capsules à un traitement de conditionnement permettant soit un développement continu soit un stockage par induction de diapause.

**Patentansprüche**

1. Verfahren zur Massenerzeugung eines insektenfressenden Insekts in einem Vermehrungskäfig, worin ausgewachsene Insekten aus einem zuvor hergestellten Inokulum, bestehend aus ersten Wirtseiern und angeordnet auf einem ersten Trägermaterial, mit zweiten Wirtseiern zusammengebracht werden, die auf einem zweiten Trägermaterial angeordnet sind, dem man zur Bildung des genannten Inokulums einen Teil entnimmt, dadurch gekennzeichnet, daß man eine konstante Population ausgewachsener Insekten aus dem genannten Inokulum, den man zuvor einer Inkubation unterzogen hat, bei konstanter Temperatur hält, daß man das genannte Inokulum in dem Käfig während eines Zeitraums aktiviert, der dem Zeitraum entspricht, der für die Entwicklung des ausgeswachsenen bei genannter Temperatur erforderlich ist, daß man das genannte erste und das genannte zweite Trägermaterial mit gleicher Geschwindigkeit kontinuierlich durch den genannten Käfig bewegt, wobei die genannte Geschwindigkeit es ermöglicht, während eine festgelegten Einwirkzeit die zweiten Wirtseier, die von dem Insekt aus dem genannten Inokulum befallen werden sollen, kontinuierlich und regelmäßig der Wirkung auszusetzen, wobei das genannte Insekt ernährt wird, daß man dann die so befallenen zweiten Wirtseier kontinuierlich einsammelt und diesen die Fraktion zur Bildung des Inokulums in Form des genannten ersten Trägermaterials nach vorheriger Inkubation entnimmt.

2. Verfahren nach Anspruch 1, dadurch gekennzeichnet, daß es sich bei dem insektenfressenden Insekt um Trichogramma maïdis und bei dem Wirtsei um dasjenige von Ephestia kuehniella handelt, daß die Käfigtemperatur 22°C und die Einwirkzeit 24 Stunden sowie die Inokulum-Aktivierungszeit fünf Tage beträgt.

3. Vorrichtung für die Anwendung des Verfahrens nach einem der Ansprüche 1 oder 2, bestehend aus einem beleuchteten und temperaturgeregelten Gehäuse, welches einen Populationskäfig (7) bildet, dadurch gekennzeichnet, daß das erste Trägermaterial für das Inokulum aus einem schmalen Transportband (A) besteht, während des zweite Trägermaterial, welches für die zu befallenden Wirtseier bestimmt ist, aus einem breiten Transportband (B) besteht, daß der Käfig einen Einlauf (11) für genannte breite Band (B) und eine Aufnahme (8) für die Aktivierung des Inokulums (A) sowie einen Auslauf (19) für das genannte breite Band (B) und für das Aufwickeln einer schmalen Spiralwindung (2), die der Bildung des genannten Inokulums dienen soll, enthält, wobei dei genannten Transportbänder auf Rollen (12) geführt und bei gleicher Geschwindigkeit kontinuierlich durch ein Motoraggregat (1) angetrieben werden, während die genannte Aufnahme (8) für die Aktivierung während der Aktivierungsdauer des Inokulums schemale Spiralwindungen (2) enthalten kann und daß der genannte Käfig außerdem mit außenliegenden Vorrichtungen zur Rückgewinnung (10) der befallenen Wirtseier und zur Aufbereitung (4, 5) des genannten breiten Bandes (B) verbunden ist.

4. Vorrichtung nach Anspruch 3, dadurch gekennzeichnet, daß die Rollen (12) aus zwei koaxialen Rollen bestehen, eine Rolle (13) für das schmale Band (A) des Inokulums und die andere Rolle (14) für das breitere Band (B) der zweiten zu befallenden Wirtseier.

5. Vorrichtung nach Ansprüche 3 oder 4, dadurch gekennzeichnet, daß das Mittel zur Rückgewinnung der befallenen Wirtseier aus einer Burstenwalze (10) zur Aufnahme des Bandes (B) besteht und daß die Mittel zur Aufbereitung des genannten Bandes ein Spülorgan (4) und ein Organ (5) zur beidseitigen Tränkung mit gesunden Wirtseiern aufweisen, dessen Auslauf mit dem Einlauf (11) des Käfigs verbunden ist, und wobei die Durchlaufzeit des genannten Bandes außerhalb des Käfigs 24 Stunden beträgt.

6. Vorrichtung nach Anspruch 5, dadurch gekennzeichnet, daß die Aufnahme (8) des Käfigs fünf Spiralwindungen (2) enthält, die als Trägermaterial für das Inokulum nach erfolgter Inkubation dienen und die dazu bestimmt sind, jeweils fünf Tage in dem Käfig zu verbleiben, wobei alle 24 Stunden eine Spirale erneuert wird.

7. Anwendung des Verfahrens nach einem der Ansprüche 1 oder 2 in der biologischen Schädlingsbekämpfung (lutte biologique), indem im Feld Kapseln abgeworfen werden, die Eier von insektenfressenden Insekten enthalten, dadurch gekennzeichnet, daß man die zweiten befallenen Wirtseier, die mit Hilfe des Verfahrens nach Anspruch 1 oder 2 erzeugt wurden, als Inokulum in Anwesenheit von gesunden Wirtseiern im Innern der Kapseln im Verhältnis 1:15 einsetzt.

8. Anwendung nach Anspruch 7, dadurch gekennzeichnet, daß man die Kapseln einer Aufbereitungsbehandlung unterzieht, die entweder eine kontinuierliche Entwicklung oder eine Konservierung durch Einleitung der Diapause ermöglicht.

**Claims**

1. Process for the bulk production of an entomophagous insect, in a multiplication cage in which adult insects, originating from a previously formed inoculum composed of parasitized first host eggs arranged on a first support, are brought into contact with second host eggs to be parasitized, arranged on a second support, from which a portion is withdrawn to form the said inoculum, which process is characterized in that a constant population of adult insects from the said inoculum which has been subjected to a prior incubation is maintained at constant temperature, in that the said inoculum is activated in the cage for a period corresponding to the time needed for emergence of the adult at the said temperature, in that the said first and second supports are passed in continuous fashion through the said cage at the same speed, the said speed enabling the second host eggs, which are to be parasitized by the insect which has emerged from the said inoculum, to be presented in a continuous and regular manner for a specified presentation time while the said insect is fed, and in that the second host eggs thus parasitized are then collected in continuous fashion, from which second host eggs the fraction forming the inoculum is withdrawn in the form of the said first support after the prior incubation.

2. Process according to Claim 1, characterized in that the entomophagous insect is Trichogramma maidis and the host egg is that of Ephestia kuehniella, the cage temperature being 22°C, the presentation time being 24 hours and the time of activation of the inoculum being five days.

3. Device for carrying out the process according to one of Claims 1 and 2, comprising an illuminated and temperature-regulated enclosure forming a population cage (7), characterized in that the first support intended for the inoculum consists of a narrow conveyor belt (A), the second support intended for the host eggs to be parasitized consisting of a broad conveyor belt (B), in that the cage comprises an entrance (11) for the said broad belt (B) and a housing (8) for activation of the inoculum (A), as well as an exit (19) for the said broad belt (B) and for winding a narrow coil (2) intended for the formation of the said inoculum, the said conveyor belts being guided on rollers (12) and driven in continuous fashion at the same speed by a motor unit (1), it being possible for the said activation housing (8) to contain narrow coils (2) during the period of activation of the inoculum, and in that the said cage is, in addition, linked to external means for recovery (10) of the parasitized host eggs and for reconditioning (4, 5) the said broad belt (B).

4. Device according to Claim 3, characterized in that the rollers (12) consist of two coaxial rollers, one (13) for the narrow belt (A) for the inoculum, the other (14) for the broader belt (B) for the second host eggs to be parasitized.

5. Device according to Claims 3 and 4, characterized in that the means for recovery of the parasitized host eggs consists of a brush roller (10) receiving the belt (B), and in that the means for reconditioning the said belt comprise a rinsing member (4) and a member (5) for sticking healthy host eggs onto both of its faces, the exit of which is connected to the entrance (11) of the cage, the time during which the said belt travels outside the cage being 24 hours.

6. Device according to Claim 5, characterized in that the housing (8) of the cage contains five coils (2) for support of the inoculum which has undergone the prior incubation, and which are designed to spend five days each in the cage, with the renewal of a coil every 24 hours.

7. Application of the process according to one of Claims 1 and 2 to biological control by releasing in the field capsules containing entomophagous insect eggs, characterized in that the parasitized second host eggs obtained by the process according to Claim 1 or 2 are employed as an inoculum in the presence of healthy host eggs inside capsules, in the ratio 1:15.

8. Application according to Claim 7, characterized in that the capsules are subjected to a conditioning treatment permitting either continuous development or storage by the induction of diapause.

# FIG. 1

# FIG. 2

FIG.3

FIG.4

FIG.5

FIG.6

FIG.7

3